(19) Europäisches Patentamt
European Patent Office
Office européen des brevets

(11) Veröffentlichungsnummer: **0 299 296 B1**

(12) **EUROPÄISCHE PATENTSCHRIFT**

(45) Veröffentlichungstag der Patentschrift: **23.01.91**

(51) Int. Cl.$^5$: **G 03 C 7/42,** G 03 C 5/44, C 07 D 251/08

(21) Anmeldenummer: **88110530.8**

(22) Anmeldetag: **01.07.88**

(54) **Bleichbäder mit bleichbeschleunigenden Substanzen.**

(30) Priorität: **14.07.87 DE 3723195**

(43) Veröffentlichungstag der Anmeldung: **18.01.89 Patentblatt 89/03**

(45) Bekanntmachung des Hinweises auf die Patenterteilung: **23.01.91 Patentblatt 91/04**

(84) Benannte Vertragsstaaten: **BE DE FR GB IT**

(56) Entgegenhaltungen:
**EP-A-0 151 305**
**FR-A-2 259 537**
**GB-A-1 150 466**

(73) Patentinhaber: **Agfa-Gevaert AG**
**D-5090 Leverkusen 1 (DE)**

(72) Erfinder: **Bergthaller, Peter, Dr.**
**Leuchter Gemark 5a**
**D-5060 Bergisch Gladbach 2 (DE)**
Erfinder: **Berthold, Werner, Dr.**
**von-Böttinger-Strasse 13**
**D-5090 Leverkusen (DE)**
Erfinder: **Häseler, Helmut**
**Fichtestrasse 80**
**D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 299 296 B1

**Beschreibung**

Die Erfindung betrifft Bleichbäder zur Verarbeitung eines belichteten farbfotografischen lichtempfindlichen Silberhalogenidmaterials, bei dem die Bleichfunktion beschleunigt ist, wodurch die Verarbeitungszeit abgekürzt wird und bei Umkehrmaterialien die Minimaldichten verringert werden.

Die grundlegenden Stufen der Verarbeitung von lichtempfindlichen Farbmaterialien umfassen allgemein eine Farbentwicklungsstufe und eine Silber-Entfernungsstufe. Bei Umkehrmaterialien kommen noch eine vorgeschltete Schwarz/Weiß-Entwicklung und eine Zweitbelichtung hinzu. In der letzten Stufe wird das bei der Entwicklung erzeugte Silber mit einem Bleichmittel oxidiert und mit einem Fixiermittel gelöst.

Die Entfernung des Silbers kann zweistufig mit einem Bleichbad und einem Fixierbad oder einstufig mit einem Bleich-Fixier-Bad durchgeführt werden.

Die Bleichverarbeitung wird überwiegend unter Verwendung eines Eisen(III)-ionenkomplexsalzes (zum Beispiel Aminopolycarbonsäure-Eisen(III)-komplexsalz, insbesondere Eisen(III)-ethylendiamintetraacetat-komplexsalz) durchgeführt. Auch Iodosoverbindungen und Persulfate sind geeignet.

Jedoch weisen Eisen(III)-ionenkomplexsalze eine vergleichsweise geringe Oxidationskraft auf und haben daher insbesondere bei Umkehrmaterialien eine unzurreichende Bleichkraft. Vor allem bei Umkehrmaterialien bestand daher ein Bedürfnis, die Bleichkraft einer Bleichlösung oder Bleich-Fixier-Lösung, die ein Bleichmittel mit geringer Bleichkraft, insbesondere ein Eisen(III)-ionkomplexsalz enthält, zu erhöhen.

Um die Bleichkraft einer Bleichlösung oder Bleich-Fixier-Lösung, die ein Eisen(III)-ionkomplexsalz wie Eisen(III)-ethylendiamintetraacetat als Bleichmittel enthält, zu erhöhen wurde vorgeschlagen, verschiedene Bleichbeschleuniger dem Verarbeitungsbad zuzusetzen.

Beispiele für solche Bleichbeschleuniger umfassen Thioharnstoffderivate, (JP—OS 8506/70, US—PS 3 706 561), Selenoharnstoffderivate (JP—OS 280/71), Mercaptoverbindungen mit fünfgliedrigem Ring (GB—PS 1 138 842) und Thioharnstoffderivate, Thiazolderivate und Thiadiazolderivate (CH—PS 336 257). Außerdem werden 5-Mercaptotetrazole als beschleunigende Mittel für die Entfernung von Silber in einer Bleich-Fixier-Lösung verwendet (GB—PS 1 138 842). Jedoch weisen diese Verbindungen eine schwache beschleunigende Kraft, geringe Löslichkeit oder mangelnde Stabilität in der Verarbeitungslösung auf.

DE—OS 3 518 257 beschreibt die bleichbeschleunigende Wirkung von Verbindungen der Formeln

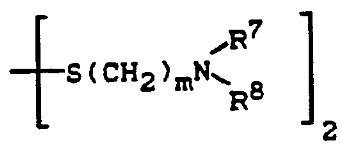

bzw.

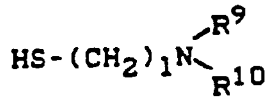

wobei $R^7$ bis $R^{10}$ Wasserstoff, Alkyl, Acyl oder gemeinsam die restlichen Glieder eines Ringes, m und l eine ganze Zahl 1 bis 3 bedeuten.

Auch diese Vrbindungen zeigen nur eine mäßige bleichbeschleunigende Wirkung und sind für Color-Umkehrmaterialien, insbesondere für Color-Umkehrpapier wenig geeignet.

Bei der Colorumkehrpapier-Verarbeitung werden nach der Erstentwicklung, der Zweitbelichtung und der Farbentwicklung zur Silberentfernung aus dem Papier üblicherweise Bleichfixierbäder auf Basis Eisen-II-EDTA benutzt. Im Gegensatz zur Colornegativpapier-Verarbeitung sind hierbei Zusätze von Bleichbeschleunigern zum Bleichfixierbad erforderlich, um das Bleichen ausreichend schnell und vollständig zu bewirken. Dazu werden Verbindungen wie 3-Mercapto-1,2,4-triazol oder 5-Amino-2-mercapto-1,3,4-thiodiazol mit Erfolg benutzt.

Aber auch bei Colornegativpapier hat sich der Einsatz von Bleichbeschleunigern im Bleichfixierbad als zweckmäßig erwiesen. Bei unzureichendem Bleichen verbleiben Restsilbermengen in den Materialien, was bei dem meist IR-gesteuerten Schneidegerät zu Fehlinformationen führen kann, durch die der Bildstreifen nicht zwischen den Bildern sonern mitten durch die Bilder geschnitten wird.

Benutzt man statt eines Bleichfixierbades zwei getrennte Bäder, nämlich Bleichbad und Fixierbad, was wegen der leichteren Rückgewinnungsmöglichkeit des gelösten Silbers vorteilhaft sein kann, lassen sich diese Bleichbeschleuniger nicht benutzen, weil sie keine ausreichende, beschleunigende Wirkung mehr haben, und weil sie teilweise nicht ausreichend löslich sind. Ohne Bleichbeschleuniger zeigen die unzureichend gebleichten Photopapiere in den dunklen Bildpartien erhebliche Restsilbergehalte.

Bei der Colorumkehrpapier-Verarbeitung ist üblicherweise in den Verarbeitungsmaschinen die Zahl der Chemikalien- und Wassertanks begrenzt. Bei getrenntem Bleichen und Fixieren erhöht sich die erforderliche Tankzahl ohnehin um 2, eine zusätzliches Conditionierbad, wie es beim Umkehrfilm gebräuchlich ist, weil sich das üblichwerweie verwendetet Conditioniermittel Thioglyzerin im Bleichbad

sehr schnell zersetzt, würde sie um 3 Tanks erhöhen. Das ist in vielen Fällen ohne kostpielige Maschinenumbauten nicht möglich. Um die Zahl der Tanks möglichst gering zu halten, ist man deshalb bestrebt, ein zusätzliches Conditionierbad zu vermeiden.

Es besteht deshalb Interesse an Zusätzen zu Bleichbädern, die so stark bleichbeschleunigend wirken, daß innerhalb der üblichen Bleichzeiten (1 bis 5 min) auf den Fotopapieren reine Weißen entstehen, und die zudem ausreichend beständig in den üblichen Eisen-III-EDTA-Bleichbädern sind.

Aufgabe der vorliegenden Erfindung ist die Bereitstellung von Bleich- bzw. Bleichfixierbädern die stabil sind und eine ausgezwichnete Bleichgeschwindigkeit aufweisen. Vor allem sollen sie bei Umkehrmaterialien bei den üblichen Verarbeitungszeiten für eine befriedigende Bleichung sorgen. Dabei sollen andere photographische Eigenschaften nicht verschlechtert werden.

Die Aufgabe wird dadurch gelöst, daß man Bleich- oder Bleichfixierbädern, die ein Eisen(III)-ionenkomplexsalz als Bleichmittel enthalten, eine Verbindung der allgemeinen Formel

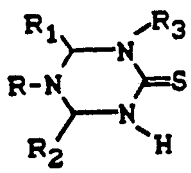

zusetzt, in der

R Alkyl oder eine Gruppe $(L)_n$—Z—,

$R_1$ und $R_2$ unabhängig voneinander Wasserstoff oder $C_1$—$C_4$-Alkyl,

$R_3$ Wasserstoff oder gegebenenfalls mit wasserlöslich machenden Gruppe substituiertes $C_1$—$C_4$-Alkyl oder $C_2$—$C_4$-Alkenyl,

L eine wasserlöslich machende Gruppe,

Z ein Zwischenglied und

n 1 oder bedeuten.

Bevorzugt bedeuten $R_1$ und $R_2$ Wasserstoff, Methyl oder Ethyl, R eine Gruppe $(L_1)_n$—$Z_1$—, worin $Z_1$, $C_1$—$C_4$-Alkylen oder Phenylen und $L_1$ einen Sulfonsäure-, Carbonsäure-, Phosphonsäure- oder Phosphorsäurerest bedeuten, und $R_3$ Wasserstoff, $C_2$—$C_4$-Alkenyl oder gegebenenfalls durch Carboxy oder Sulfo substituiertes $C_1$—$C_4$-Alkyl.

Geeignete Verbindungen sind z.B.

I 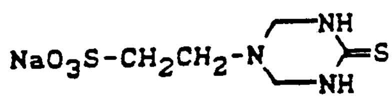

II 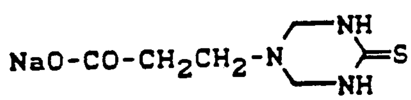

III 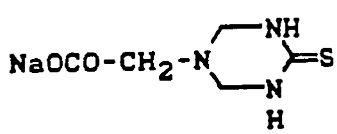

IV 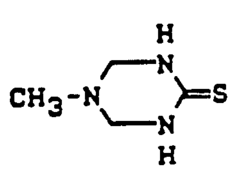

V 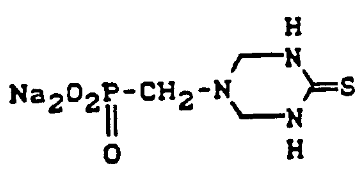

VI 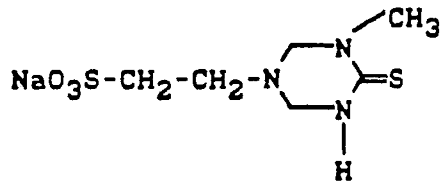

VII 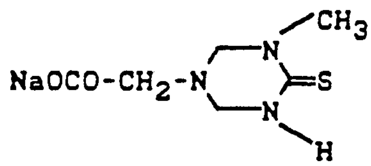

VIII 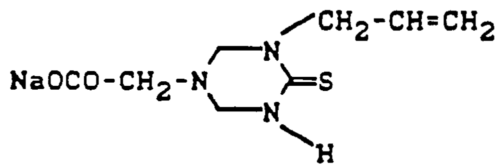

IX 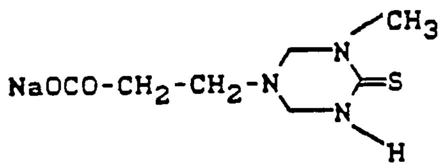

X 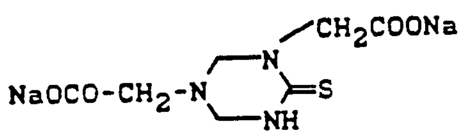

XI 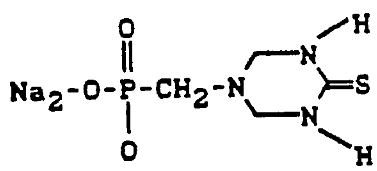

XII 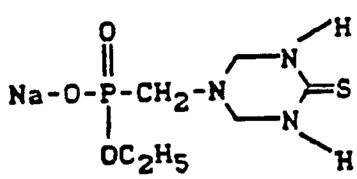

XIII 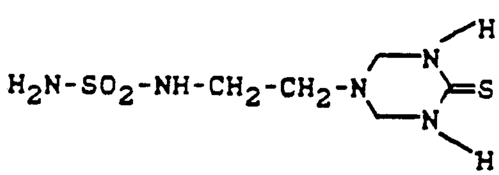

XIV 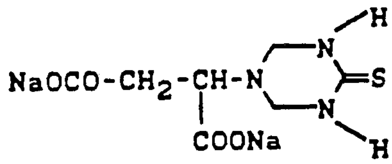

XV 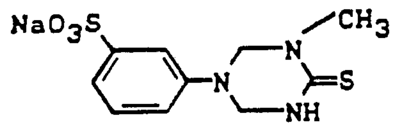

Die erfindungsgemäß zu verwendenden Verbindungen werden aus einem Thioharnstoff der Formel

$$R_3—NH—CS—NH_2,$$

der annähernd gleichen molaren Menge eines Amins der Formel

$$R—NH_2$$

und der annähernd doppelt molaren Menge eines Aldehyds der Formel

$$R_1—CHO \text{ bzw. } R_2—CHO$$

wobei R, $R_1$, $R_2$ und $R_3$ die vorstehend angegebene Bedeutung besitzen, in wäßrigem Alkali hergestellt; zur Erläuterung wird nachstehend die Synthese der Verbindung VII beschrieben. Die anderen Verbindungen werden analog hergestellt.

Synthese der Verbindung VII:

Man trägt in 65 ml Wasser ohne Kühlung ein:
45 g (0,6 Mol) Glykokoll
24 g (0,6 Mol) Natriumhydroxid
45 g (0,5 Mol) N-Methylthioharnstoff
120 g (1,2 Mol) einer 30 gew.-%igen wäßrigen Formaldehydlösung.

Die Temperatur steigt auf 70°C und wird noch 30 Min. auf 70°C gehalten. Nach Abkühlen auf Raumtemperatur kristallisiert das Produkt aus.

Man digieriert mit 1000 ml Aceton, saugt ab und trocknet an der Luft.

Ausbeute 180 g. Schmelzpunkt 73—78°C unter Wasseraustritt, nach Wiedererstarren ab 100°C Zersetzung bei 228°C.

Kernresonanzspektrum: in $D_2O$
$CH_3$: bei $\delta = 3,2$ ppM
$CH_2$: bei $\delta = 3,34$, 4,24 und 4,36
$H_2O$: 4,72

Die Menge der erfindungsgemäßen Verbindung in den Bleich- und Bleichfixierbädern variiet je nach der Art der Verarbeitungslösung, der Art des zu verarbeitenden photographischen Materials, der Verarbeitungstemperatur, der zur Durchführung der gewünschten Verarbeitung benötigten Zeit, usw. Jedoch ist eine Menge von $1 \times 10^{-5}$ bis 1 Mol pro Liter Bleich- bzw. Bleichfixierbad geeignet, wobei $1 \times 10^{-3}$ bis $1 \times 10^{-1}$ Mol bevorzugt sind. Im allgemeinen wird mit einer geringen Menge ein geringer Bleichbeschleunigungseffekt erzeilt, hingegen besteht mit einer größeren als der benötigten Menge die Gefahr einer Ausfällung, die zu Störungen bei der Verarbeitung führen kann. Daher wird der jeweils beste Bereich durch einfache Vorversuche bestimmt. Die erfindungsgemäß zu verwendende Verbindung kann dem Bleich- oder Bleichfixierbad direkt zugesetzt oder durch ein vorgeschaltetes Conditionierbad eingebracht werden.

Als Eisen(III)-ionenkomplexsalze eignen sich Komplexe von Eisen(III)-ionen und einem chelatbildenden Mittel wie einer Aminopolycarbonsäure, einer Aminopolyphosphonsäure oder einem Salz davon, insbesondere einem Alkalimetallsalz oder Ammoniumsalz.

Typische Beispiele chelatbildender Mittel sind Ethylendiamintetraessigsäure; Dinatriumethylendiamintetraacetat; Diammoniumethylendiamintetraacetat; Tetra(trimethylammonium)-ethylendiamintetraacetat; Tetrakaliumethylendiamintetraacetat; Tetranatriumethylendiamintetraacetat; Trinatriumethylendiamintetraacetat; Diethylentriaminpentaessigsäure; Pentanatriumdiethylentriaminpentaacetat; Ethylendiamin-N-(β-hydroxyethyl)-N,N',N'-triessigsäure; Trinatriumethylendiamin-N-(β-hydroxyethyl)-N,N',N'-triacetat; Triammoniumethylendiamin-N-(β-hydroxyethyl)-N,N',N'-triacetat; Propylendiamintetraessigsäure; Dinatriumpropylendiamintetraacetat; Nitrilotriessigsäure; Trinantriumnitrilotriacetat; Cyclohexandiamintetraessigsäure; Dinatriumcyclohexandiamintetraacetat; Nitrilotriessigsäure; Trinatriumnitrilotriacetat; Cyclohexandiamintetraessigsäure; Dinatriumcyclohexandiamintetraacetat; Iminodiessigsäure; Dihydroxyethylglycin; Ethylether-diamintetraessigsäure; Glykolether-diamintetraessigsäure; Ethylendiamintetrapropionsäure; Phenylendiamintetraessigsäure; 1,3-Diaminopropanol-N,N,N',N'-tetramethylenphosphonsäure; Ethylendiamin-N,N,N',N'-tetramethylenphosonsäure; 1,3-Propylendiamin-N,N,N',N'-tetramethylenphosphonsäure, usw.

Das Eisen(III)-ionenkomplexsalz kann in der Form des Komplexsalzes verwendet oder in situ in dem Bleich- oder Bleichfixierbad hergestellt werden. Geeignete Kationen sind Alkalikationen und Ammonium; letzteres ist, bevorzugt.

Die erfindungsgemäße Bleichlösung kann rehalogenierende Mittel wie Bromide (z.B. Kaliumbromid, Natriumbromid, Ammoniumbromid, usw.), Chloride (z.B. Kaliumchlorid, Natriumchchlorid, Ammoniumchlorid, usw.), und dergleichen zusätzlich zu den Bleichmitteln enthalten. Darüber hinaus können Zusätze, die eine pH-Pufferwirkung haben, wie anorganische Säuren, organische Säuren oder die Salze davon, die zur Verwendung in üblichen Bleichlösungen bekannt sind(z.B. Borsäure, Borax, Natriummetaborat, Essigsäure, Natriumacetat, Natriumcarbonat, Kaliumcarbonat, Phosphorige-Säure, Phosphorsäure, Natriumphosphat, Zitronensäure, Natriumzitrat, Weinsäure, usw.) zugesetzt werden.

Der pH-Wert der Bleichlösung beträgt vorzugsweise 3,0 bis 8,0, insbesondere 4,0 bis 7,0.

Bei Verwendung in einem Bleich-Fixier-Bad können übliche Fixiermittel, d.h. wasserlösliche, Silberhalogenid auflösende Mittel, wie Thiosulfat (z.B. Natriumthiosulfat, Ammoniumthiosulfat, Ammoniumnatriumthiosulfat, Kaliumthiosulfat, usw.); Thiocyanate (z.B. Natriumthiocyanat; Ammoniumthiocyanat; Kaliumthiocyanat, usw.); Thioetherverbindungen (z.B. Ethylenbisthioglykolsäure, 3,6-Dithia-1,8-octandiol, usw.); und Thioharnstoffe allein oder in einer Kombination von zwei oder mehreren verwendet werden. Zusätzlich können spezielle Bleich-Fixier-Mittel, die eine Kombination eines Fixiermittels und eine große Menge einer Halogenidverbindung wie Kaliumjodid enthalten, ebenfalls verwendet werden.

In der Bleich-Fixier-Zusammensetzung ist das Eisen(III)-ionenkomplexsalz üblicherweise in einer Menge von 0,1 bis 1 Mol/l vorhanden. Die Menge des Fixiermittels beträgt im allgemeinen 0,2 bis 4 Mol pro liter der Bleich-Fixier-Lösung.

Bleich-Fixier-Lösungen können darüber hinbaus konservierende Mittel wie Sulfite (z.B. Natriumsulfit, Kaliumsulfit, Ammoniumsulfit, usw.), Hydroxylamin, Hydrazin, Alkdehyd-Bisulfit-Addukte (z.B. Acetaldehydnatriumbisulfitaddukt), usw. enthalten. Darüber hinaus können verschiedene optische Aufheller, Entschäumungsmittel, oberflächenaktive Mittel, organische Lösungsmittel (z.B. Methanol) und bekannte Bleich-Fixier-Beschleunigungsmittel, z.B. Polyaminverbindungen. (US—PS 3 578 457), Thioharnstoffe (US—PS 3 617 283), Iodide (DE—PS 1 127 715), Polyethylenoxide (DE—PS 966 410), Stickstoff enthaltende heterocyclische Verbindungen (DE—PS 1 290 812) und andere Thioharnstoffe verwendet werden. Der pH-Wert der Bleich-Fixier-Losung liegt bei der Verwendung gewöhnlich bei 4,0 bis 9,0, besonders bevorzugt bei 5,0 bis 8,0.

## Beispiel 1

Ein Colornegativpapier wird hinter einem Stufenkeil belichtet und wie folgt verarbeitet:

| | |
|---|---|
| Farbentwickler 92 CD | 210 sec. 33°C |
| Wässerung | 20 sec. 20°C |
| Abstreifen des Wassers von der Oberfläche | |
| der Bilder Bleichbad | X sec. 20°C |
| Wässerung | 60 sec. 20°C |
| Fixierbad | 60 sec. 20°C |
| Wässerung | 120 sec. 20°C |
| Trocknen | |

Restsilberbestimmung

Die verarbeiteten Stufenkeile werden mit einem Infrarot-Silberdetector PM 8030 der Fa. Photo-Matic, Denmark untersucht. In der nachfolgenden Tabelle ist angegeben, bei welchen Bleichzeiten in den Bildschwärzen noch Silber vorhanden ist.

Beispiel 2

Beispiel 1 wird wiederholt, wobei dem Bleichbad 10 ml/l einer 1 molaren wäßrigen Lösung der Verbindung VI zugesetzt werden.

Beispiel 3

Beispiel 1 wird wiederholt, wobei dem Bleichbad 10 ml/l einer 1 molaren wäßrigen Lösung der Verbindung III zugesetzt werden.

| Beispiel | Bleichzeit X (sec) | Restsilber (ja/nein) |
|---|---|---|
| 1 | 30 | ja |
| 1 | 40 | ja |
| 1 | 50 | ja |
| 2 | 30 | ja |
| 2 | 40 | nein |
| 2 | 50 | nein |
| 3 | 30 | nein |
| 3 | 40 | nein |
| 3 | 50 | nein |

Bei einer üblichen Bleichzeit von 45 Sekunden kann mit den erfindungsgemäßen Verbindungen eine einwandfreie Bleichung erzielt werden.

Zusammensetzung der fotografischen Bäder des Beispiels 1

| | | |
|---|---|---|
| Farbentwickler: | Standardrezept 92 CD | |
| Bleichbad: | Wasser | 700 ml |
| | $NH_4$-Fe-EDTA | 65 g |
| | EDTA | 10 g |
| | $NH_4Br$ | 100 g |
| | $Na_2SO_3$ | 2g |
| | mit Essigsäure auf pH 6,0 einstellen, Auffüllen mit Wasser auf 1 Liter | |
| Fixierbad: | Ammoniumthiosulfat | 100 g |
| | Na-sulfit, sicc. | 10 g |
| | Na-disulfit | 3 g |
| | mit Wasser auffüllen auf 1 Liter | |

Beispiel 4

Die erfindungsgemäßen Substanzen werden in Mengen von 0,5—10 g pro Liter üblichen fotografischen Bleichbädern auf Eisen-III-EDTA-Basis zugegeben. Die Bleichbäder werden bei pH-Werten

zwischen 4 und 8 bei Temperaturen zwischen 25°C und 50°C mit Verarbeitungszeiten von 1 min bis 6 min benutzt.

Verwendung finden lichtempfindiche Fotomaterialien mit den üblichen Merhrschichtaufbauten für Colorumkehr-Papier-Verfahren.

Die fotografische Verarbeitung erfolgt in den Stufen:

| | | |
|---|---|---|
| Erstentwicklung | 38°C | 75 s |
| 1. Wässerung, dabei Zweitbelichtung mit mindestens 100 Lux | 35—41°C | 90 s |
| Farbentwicklung | 38°C | 135 s |
| 2. Wässerung | 25—41°C | 45 s |
| Bleichbad | 38°C | 135 s |
| 3. Wässerung | 25—41°C | 45 s |
| Fixierbad | 38°C | 60 s |
| Schlußwässerung | 25—41°C | 135 s |

Eine Wässerung zwischen Bleich- und Fixierbad ist erforderlich, damit das Fixierbad nicht mit Bleichbad verunreinigt wird, was die Entsilberung erschweren würde.

Die Verarbeitungsbäder sind wie folgt zusammengesetzt:

Erstentwickler:

| | |
|---|---|
| Hydrochinonmonosulfonat | 20 g |
| ATMP-Säure 50 gew.-% (Kalkschutzmittel) | 1,2 ml |
| DTPA | 2,5 g |
| Natriumsulfit | 24 g |
| Kaliumcarbonat | 25 g |
| Kaliumthiocyanat | 1,1 g |
| Dimethylhydroxyphenidon | 1,3 g |
| Kaliumbromid | 2,5 g |
| Kaliumiodid, 0.1 gew.-%ige wäßrige Lösung | 4 ml |

auf 1000 ml mit Wasser auffüllen
pH 9,8 einstellen

Farbentwickler:

| | |
|---|---|
| CD 3-Sulfat | 4,9 g |
| Benzylalkohol | 16 ml |
| Kaliumcarbonat | 24 g |
| Natriumsulfit | 1,7 g |
| Hydroxyethandiphosphonsäure 60 gew.-% (Kalkschutzmittel) | 0,4 ml |

| | |
|---|---|
| 2,2-Ethylendithioethanol | 0,3 g |
| $N(CH_2—COONa)_3$ (Kalkschutzmittel) | 2,2 g |
| Hydroxylaminsulfat | 2,1 g |
| Kaliumbromid | 0,5 g |
| Kaliumhydroxid | 1,2 g |
| Ethylenglykol | 15 ml |

auf 1000 ml mit Wasser auffüllen
pH 10,5 einstellen

Bleichbad:

| | |
|---|---|
| Ammonium-Eisen-III-EDTA | 66 g |
| EDTA-Säure | 12 g |
| Ammoniumbromid | 100 g |
| Natriumsulfit | 3,3 g |

auf 1000 ml Wasser auffüllen
pH 6,0 einstellen

Fixierbad:

| | |
|---|---|
| Ammoniumthiosulfat | 88 g |
| Natriumsulfit | 9 g |
| Natriumdisulfit | 1,5 g |

auf 1000 ml mit Wasser auffüllen
pH 6,6 einstellen

Die Bleichwirkung wird anhand der Bildweißen (Minimale Dichte = Grudschleier) in den 3 Grundfarben densitometrisch ausgemessen.

| Bad | D min | | |
|---|---|---|---|
| | Gelb | Purpur | Blaugrün |
| Bleichfixierbad | 0,12 | 0,11 | 0,08 |
| Bleichbad ohne erfindungsgemäßen Bleichbeschleuniger | 0,37 | 0,33 | 0,28 |
| Bleichbad + 0,2 g Substanz II/1 | 0,26 | 0,19 | 0,15 |
| Bleichbad + 0,5 g Substanz II/1 | 0,16 | 0,13 | 0,10 |
| Bleichbad + 1,0 g Substanz II/1 | 0,12 | 0,10 | 0,08 |
| Bleichbad + 0,2 g Substanz III/1 | 0,25 | 0,21 | 0,17 |
| Bleichbad + 0,5 g Substanz III/1 | 0,19 | 0,15 | 0,11 |
| Bleichbad + 1,0 g Substanz III/1 | 0,12 | 0,10 | 0,08 |

Zum Vergleich wird die für Colorumkehrpapier übliche Verarbeitung mit einem Bleichfixierbad herangezogen, das Bleichbad, 3. Wässerung und Fixierbad ersetzt und bei 38°C 120 s einwirkt.

| Bleichfixierbad: | |
|---|---|
| Ammonium-Eisen-III-EDTA | 70 g |
| Ammoniumthiosulfat | 100 g |
| Natriumdisulfit | 18 g |
| 3-Mercapto-1,2,4-triazol | 0,4 g |
| EDTA-Säure | 2,3 g |
| auf 1000 ml mit Wasser auffüllen<br>pH 6,2 einstellen | |

Die erfindungsgemäßen Substanzen können auch einen Bleichfixierbad zugesetzt werden.

**Patentansprüche**

1. Bleich- und Bleichfixierbäder, die ein Eisen(III)-ionenkomplexsalz als Bleichmittel enthalten, dadurch gekennzeichnet, daß sie zusätzlich eine Verbindung der allgemeinen Formel

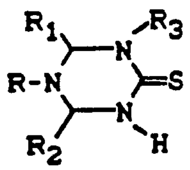

enthalten, in der
R Alkyl oder eine Gruppe $(L)_n$—Z—,
$R_1$ und $R_2$ unabhängig voneinander Wasserstoff oder $C_1$—$C_4$-Alkyl,
$R_3$ Wasserstoff, $C_2$—$C_4$-Alkenyl oder gegebenenfalls durch eine wasserlöslich machende Gruppe substituiertes $C_1$—$C_4$-Alkyl,
L eine wasserlöslich machende Gruppe,
Z ein Zwischenglied und
n 1 oder 2 bedeuten.

2. Bleich- und Bleichfixierbäder gemäß Anspruch 1, wobei $R_1$, $R_2$ Wasserstoff, Methyl oder Ethyl, R eine Gruppe $(L_1)_n$—$Z_1$—, worin $Z_1$, $C_1$—$C_4$-Alkylen oder Phenylen und $L_1$ einen Sulfonsäure-, Carbonsäure- oder Phosphorsäurerest bedeuten, und $R_3$ Wasserstoff, $C_2$—$C_4$-Alkenyl oder gegebenenfalls durch Carboxy oder Sulfo substituiertes $C_1$—$C_4$-Alkyl bedeuten.

3. Bleich- und Bleichfixierbäder gemäß Anspruch 1, wobei die zusätzlich Verbindung in einer Menge von $1 \times 10^{-5}$ bis 1 Mol pro Liter Bleich- bzw. Bleichfixierbad eingesetzt wird.

4. Bleich- und Bleichfixierbäder gemäß Anspruch 1, wobei das Eisen(III)-ionkomplexsalz der Ethylendiamintetraessigsäure als Bleichmittel verwendet wird.

**Revendications**

1. Bains de blanchiment et de blanchiment-fixation, qui contiennent comme agent de blanchiment un sel complexe d'ions de fer (III), caractérisés en ce qu'ils contiennent, en outre, un composé de la formule générale

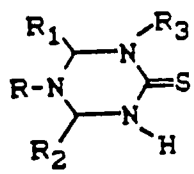

dans laquelle
R représente un radical alkyle ou un groupe $(L)_n$—Z—,
$R_1$ et $R_2$ représentent indépendamment l'un de l'autre de l'hydrogène ou un radical alkyle en $C_1$—$C_4$,

$R_3$ représente de l'hydrogène, un radical alcényle en $C_2$—$C_4$ ou un radical alkyle en $C_1$—$C_4$, substitué éventuellement par un groupe hydrosolubilisant,

L représente un groupe hydrosolubilisant,

Z représente un élément intermédiaire, et

n signifie 1 ou 2.

2. Bains de blanchiment et de blanchiment-fixation, selon la revendication 1, dans lesquels $R_1$ et $R_2$ représentent de l'hydrogène, un radical méthyle ou éthyle, R un groupe $(L_1)_n$—$Z_1$—, dans lequel $Z_1$ représente un radical alkylène en $C_1$—$C_4$ ou un radical phénylène et $L_1$ un reste d'acide sulfonique, d'acide carboxylique ou d'acide phosphorique et $R_3$ représente de l'hydrogène, un radical alcényle en $C_2$—$C_4$ ou un radical alkyle en $C_1$—$C_4$, éventuellement substitué par un radical carboxy ou sulfo.

3. Bains de blanchiment et de blanchiment-fixation selon la revendication 1, dans lesquels le composé supplémentaire est ajouté en quantités de $1 \times 10^{-5}$ à 1 mole par litre de bain de blanchiment ou de bain de blanchiment-fixation.

4. Bains de blanchiment et de blanchiment-fixation, selon la revendication 1, dans lesquels le sel complexe d'ions de fer (III) de l'acide éthylène-diamine-tétraacétique est utilisé comme agent de blanchiment.

**Claims**

1. Bleaching and bleaching/fixing baths containing an iron(III) ion complex salt as bleaching agent, characterized in that they additionally contain a compound corresponding to the general formula

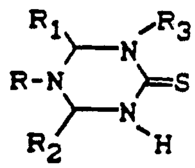

in which

R is alkyl or a group $(L)_n$—Z—,

$R_1$ and $R_2$ independently of one another represent hydrogen or $C_{1-4}$ alkyl,

$R_3$ represents hydrogen, $C_{2-4}$ alkenyl or $C_{1-4}$ alkyl optionally substituted by a water-solubilizing group,

L is a water-solubilizing group,

Z is an intermediate link and

n is 1 or 2.

2. Bleaching and bleaching/fixing baths as claimed in claim 1, wherein $R_1$ and $R_2$ are hydrogen, mthyl or ethyl, R is a group $(L_1)_n$—$Z_1$—, where $Z_1$ is $C_{1-4}$ alkylene or phenylene and $L_1$ is a sulfonic acid, carboxylic acid or phosphoric acid residue and $R_3$ is hydrogen, $C_{2-4}$ alkenyl or optionally carboxy- or sulfo-substituted $C_{1-4}$ alkyl.

3. Bleaching and bleaching/fixing baths as claimed in claim 1, wherein the additional compound is used in a quantity of $1 \times 10^{-5}$ to 1 mol per liter bleaching or bleaching/fixing bath.

4. Bleaching and bleaching/fixing baths as claimed in claim 1, wherein the iron(III) ion complex salt of ethylenediamine tetraacetic acid is used as the bleaching agent.